# EUROPEAN PATENT APPLICATION

(11) **EP 2 633 848 A1**
(43) Date of publication of application: **04.09.2013**
(21) Application number: 12460048.7
(22) Date of filing: 06.08.2012
(51) Int. Cl.: A61K 8/03, A61K 8/97, A61Q 19/08

(54) **Natural multiphase cosmetics**

(30) Priority: 22.02.2012 PL 39819712
(71) Applicant: "Pulanna" sp. z o.o., 24-130 Konskowola (PL)
(72) Inventor: Skwarek, Miroslaw, 24-130 Stara Wies (PL); Skwarek, Michael, 130 Stara Wies (PL)
(74) Representative: Dziubinska, Jolanta

(57) **Abstract**

The subject of the invention is natural two-phase cosmetics in the gel form with the emulsion rich in various natural components suspended inside, intended especially for skin and hair care.

In the solution according to the invention, in the natural gelling substance made from thickening agents in the form of gelatine, xanthan gum, sucrose esters, used in the amount of 50.0%, 30.0%, and 20.0%, respectively, there is suspended high-viscous emulsion containing at least 2% of the root extract of *Belamcanda chinensis,* and in the emulsion there are also suspended other oxidation-prone natural active substances.

A natural cosmetic may contain agar-agar, instead of gelatine, in the amount of 60% in the gelling mixture, and the share of sucrose esters in the mixture of gelling substances shall total 10%.

## Description

The subject of the application is natural multiphase cosmetics in the gel form with emulsion rich in diverse natural components suspended inside, intended especially for skin and hair care. There are known cosmetic gels based on synthetic gelling substances. There is also known the use of mineral modifiers of viscosity, such as bentonites and hectorites. There are generally used and known derivatives of cellulose (carboxymethylcellulose (CMC), hydroxyethylcellulose (HEC), methyl cellulose (MC) and acrylic derivatives (Acrylates Crosspolymer). Being chemically modified, the substances cannot be used in natural cosmetics intended for certification by international certifying units, such as Cosmos, Natrue or BDIH.

There is also known the use of natural gelling substances, such as agar-agar, guar gum, carrageenan, tragacanth, or xanthan gum. There are significant restriction concerning the application of these substances for the manufacture of natural cosmetics. The restrictions are caused by pH value at which they can be used and at which they retain their gelling properties. When manufacturing natural cosmetics, the kind of used preservatives should also be considered. Passed as fit for the manufacture of natural cosmetics, derivatives of sorbic and benzoic acid, as well as of benzyl alcohol, are active only at low pH level. However, several natural gelling substances require pH close to 7 in order to retain their properties. It should be emphasized that natural gums and thickening agents are excellent media for bacteria and the proper way of their preservation is fundamental for the safety of a cosmetic in microbiological aspect.

There is known the use of extracts of *Belamcanda chinensis* in pharmaceutical preparations intended for the application in various internal disorders, as well as in preparations for external application on the skin and the hair.

According to the invention, natural multiphase cosmetics contain the suspended high-viscous emulsion, containing at least 2% of the extract of *Belamcanda chinensis* root, in the natural gelling substance made of thickening agents in the form of gelatine, xanthan gum, sucrose esters, applied in the volume of 50%, 30%, and 20%, respectively; and the high-viscous emulsion contains also other oxidation-prone natural active substances.

According to the invention, natural cosmetics may contain agar, instead of gelatine, in the gelling mixture; and the participation of sucrose esters in the gelling mixture is reduced to 10%.

In the solution according to the invention a special mixture of substances of natural origin has been developed, which makes up a slightly opalescent gel, stable in time and microbiologically resistant due to various gelling mechanisms and synergy of its components. The novelty of the solution according to the invention is the application of a specially developed mixture of natural gelling substances for conditioning cosmetics, in particular, for their manufacture in multiphase form.

The essence of the solution according to the invention is the application of the extract from *Belamcanda chinensis* rhizome, both of the active substance intended for human skin care, and for the protection and stabilization of the form of the cosmetic itself. Isoflavones contained in *Belamcanda chinensis* (mainly tectorigenin) as an active substance have anti-aging properties for skin. The anti-aging mechanism is connected with phytoestrogen character of the system of isoflavones isolated from the root of *Belamcanda chinensis.* Antioxidant effect is another desirable property of the system of isoflavones from *Belamcanda chinensis.* Due to these properties, at proper concentrations of the extract (at least 2%), it is possible to use unsaturated fatty acids, vitamins and other oxidation-prone natural active substances in multiphase cosmetics. In the solution according to the invention, hyaluronic acid, collagen, or unsaturated fatty acids are used, among others, as other oxidation-prone natural active substances. In the tests run by the company the stabilizing effect of the extract from *Belamcanda chinensis* on multiphase systems of cosmetics has been confirmed. Stability tests have proved that cosmetics containing at least 2% of the extract from *Belamcanda chinensis* do not show any essential changes of physical and chemical parameters in the course of three-month shuttle test. This shows the stability of the system during at least three years of its storage in standard conditions. This result is unusual, both in the group of tested cosmetics and in the wider understanding of multiphase natural cosmetics. It can be explained with a proper selection of components of the gelling mixture and stabilizing and antioxidant effect of components included in the extract from *Belamcanda chinensis.*

When the extract from *Belamcanda chinensis* has been used in cosmetics preserved with the mixture of sorbic acid, benzyl alcohol and benzoates, unexpectedly a strong synergic effect has been observed. In antibacterial tests made for model strains of Staphylococcus aureus, Candida albicans, Pseudomonas aeruginosa and Escherichia coli, a significant (almost twofold) increase of the area of growth inhibition has been observed. In tests aimed at the optimization of preserving systems of cosmetics containing 2% of extracts from *Belamcanda chinensis,* the possibility of the reduction of the concentration of the used preservatives, with retaining full efficiency confirmed in the so-called challenge tests, has been noticed. Synergic properties have been found exclusively in relation to the above mentioned extract and preserving system. Other flavonoid extracts (Japanese Sophora and hop extract have been used for comparative studies) have had no such properties.

The above described mixture consists of gelatine used in the amount of about 50%, xantham gum used in the amount of about 30%, and about 20% of sucrose esters.

Depending on needs, gelatine may be substituted with edible agar-agar; however, the obtained product becomes more compact and fragile. Multiphase systems obtained in the second case are characterized by better presentation and durability, yet to the detriment of application properties. In the solution according to the invention, in the mixture of natural thickening agents making up the colourless gel, the high-viscous emulsion containing natural active substances, among others, the extract from *Belamcanda chinensis* rhizome, has been suspended. The high-viscous emulsion may be suspended in the gel in various geometrical forms, e.g., in the form of a flower or a fragment of DNA, making a multiphase system. The below photo shows an exemplary appearance of cosmetic preparations being the subject of the application. The form of cosmetics shown in the photo is protected in the category of the industrial design.

The solution according to the invention has been presented in examples of embodiment without limiting its application.

### Example 1

In the solution according to the invention a gel fraction and an emulsion fraction are prepared. The high-viscous emulsion with active components in various geometrical forms is suspended in the gel fraction.

The gel fraction contains the following:
- water 92%,
- the gelling mixture, applied in the amount of 5.00%, made of:
   Agar-agar in the amount of 60%,
   xantham gum in the amount of 30%,
   sucrose ester in the amount of 10%,
- the preserving mixture made from sorbic acid, benzyl alcohol, sodium benzoate, applied in the amount of 2.0%,
- excipients (pH regulators, colourants, flavours), applied in the amount of 1.0%.

According to INCI nomenclature, the emulsion fraction contains the following components:
Isohexadecane 5%
Etyhexylstearate 8%
Decyl Oleate 6%
Sorbitan Oleate (and) Polyglyceryl-3-polyricinooleate 5%
Sucrose Polystearate 1%
Aqua 66.85%
Belamcandae extract 2%
Fragrance 0.2%
Preservative 1%

The gel fraction is placed in a unit packaging, and simultaneously the high-viscous emulsion is suspended in the gel fraction in the form of spirally arranged drops, making up a multiphase system of the emulsion suspended in the gel.
The natural multiphase cosmetic is intended for face care.

### Example 2

Acting as in example 1, the gel fraction contains the following:
- water 92%,
- the gelling mixture, applied in the amount of 5.00%, containing gelatine in the amount of 50%, xanthan gum in the amount of 30%, sucrose esters in the amount of 20%,
- the preserving mixture containing sorbic acid, benzyl alcohol, sodium benzoate, applied in the amount of 2.0%,
- excipients, such as pH regulators, colourants, flavours, applied in the amount of 1.0%.

According to INCI nomenclature, the emulsion fraction contains the following components:
Isohexadecane 4%
Etyhexylstearate 6%
Decyl Oleate 4%
Sorbitan Oleate (and) Polyglyceryl-3-polyricinooleate 4%
Sucrose Polystearate 1%
Aqua 66.85%
Belamcandae extract 2%
Makadamia Ternifolia Seed Oil 2%
Persea Gratissima Oil 2%
Prunus Amygdalus Dulcis Oil 2%
Fragrance 0,2%
Preservative 1%

The natural multiphase cosmetic is intended for body care.

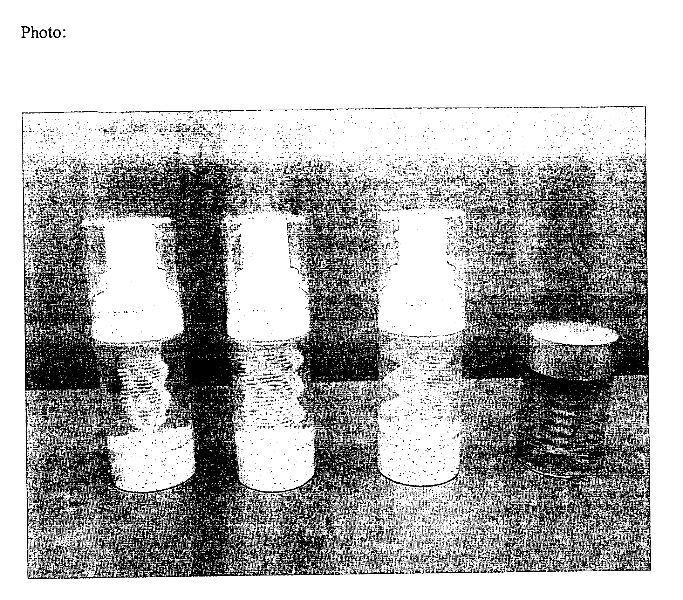

## Claims

1. Natural multiphase cosmetics containing the emulsion suspended in the gel substance, **characterized by** the fact that in the natural gelling substance made from thickening agents in the form of gelatine, xanthan gum, sucrose esters, used in the amount of 50.0%, 30.0%, and 20.0%, respectively, there is suspended high- viscous emulsion containing at least 2% of the root extract of *Belamcanda chinensis,* and in the emulsion there are also suspended other oxidation-prone natural active substances.

2. A natural cosmetic according to claim 1, **characterized by** the fact that in the gelling mixture agar-agar in the amount of 60% is used instead of gelatine, and the share of sucrose esters in the gelling mixture is proportionally reduced to 10%.
